(19) Europäisches Patentamt / European Patent Office / Office européen des brevets

(11) **EP 1 254 595 B1**

(12) **EUROPEAN PATENT SPECIFICATION**

(45) Date of publication and mention
of the grant of the patent:
**06.04.2011 Bulletin 2011/14**

(51) Int Cl.:
*G01N 1/10* (2006.01)     *G01N 1/20* (2006.01)
*A01J 5/04* (2006.01)

(21) Application number: **02009050.2**

(22) Date of filing: **23.04.2002**

(54) **Liquid sampling device**

System zur Entnahme flüssiger Proben

Système d'échantillonage pour un liquide

(84) Designated Contracting States:
**AT BE CH CY DE DK ES FI FR GB GR IE IT LI LU
MC NL PT SE TR**
Designated Extension States:
**LV MK RO SI**

(30) Priority: **24.04.2001 IL 14278101**

(43) Date of publication of application:
**06.11.2002 Bulletin 2002/45**

(73) Proprietor: **S.A.E. Afikim Milking Systems
Agricultural
Cooperative Ltd.
15148 Doar-Na Emek HaYarden (IL)**

(72) Inventor: **Kapitulskiy, Alex
Afula 18771 (IL)**

(74) Representative: **Henkel, Feiler & Hänzel
Patentanwälte
Maximiliansplatz 21
80333 München (DE)**

(56) References cited:
**DE-A1- 3 938 076      DE-U1- 9 216 200
DE-U1- 29 718 049      US-A- 3 803 921
US-A- 4 442 720**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present invention relates to methods and devices according to the preamble portion of claim 1 and claim 23, respectively, for sampling of a continuously or intermittently circulating liquid, particularly a foaming or other non-homogenous liquid, such as milk.

### BACKGROUND OF THE INVENTION

In the dairy industry milk produced from each individual cow may be sampled to check *inter alia*, for quantity of milk produced; quality of milk produced, *e.g.*, fat content, protein content, calcium content, lactose content, levels of cells such as converter cells, levels of hormones such as progesterone, and contamination or infection of the milk. It is preferable to be able to sample milk from each cow independently before the milk is merged with milk from other cows, so if any of the measured parameters are not satisfactory, the milk from specified animals only can be excluded.

Milk is characteristic of many biological liquids in that it is not homogenous. For example, fats due to their molecular weight, are less dense than water and rise to the surface of the liquid. Non-continuous sampling from any point of such a phase separated liquid can not provide accurate information about the overall composition of the liquid. Furthermore, milk flow is not generally homogenous over time. Typically the milk at the beginning of the milking session has a lower fat content than that towards the middle of the milking. Therefore non-continuous sampling at any one time point of such a non-homogenous flow of liquid can not provide accurate information about the overall composition of the liquid.

Milk is typically collected by means of vacuum action. Various milk sampling devices have been described in the prior art. US Patent No. 4,147,062 to Jaeger describes sampling apparatus in which a milk sample is collected from the milk line by sampling apparatus comprising a plunger and a pneumatic cylinder. US Patent No. 4,574,630 to Icking *et al*. describes a sampler for a milk volumeter. Milk is separated from the milk line into a separation chamber and thence into the measuring chamber of the volumeter, from where a milk sample is collected by means of a partial vacuum. US Patents Nos. 5,388,549 and 5,572,946, both to Holroyd, describe milk sampling apparatus in which a portion of the milk in the milk line is diverted through a by-pass to a collection chamber where timed milk samples are collected by means of a vacuum, using a timer, a pump and a sampling valve. The above described devices all collect whole samples of milk, at distinct times during the milk flow, and as such are not typical of the overall composition of the milk.

US-A-3803921 discloses a liquid sampling device on which the preamble portion of claims 1 and 23 is based. This device comprises a pipe section through which the liquid flows and a probe extending diametrically into the flow stream from a fitting portion. The probe has an outer conduit with plural lateral openings arranged along the length of the conduit for sampling the stream into an internal plenum and an internal conduit which is coaxial with the outer conduit which has a lateral opening at the center of the plenum through which a sample is picked up and guided to the fitting.

### SUMMARY OF THE INVENTION

The present invention seeks to provide a new device and method for sampling a flowing liquid; in which the volume of the liquid sampled is substantially proportional to the flow rate of the liquid. The sample composition is essentially representative of the composition of the flowing liquid at the time of collection.

There is thus provided in accordance with the present invention, a liquid sampling device as defined in claim 1 and a method of essentially continuously sampling a liquid flow as defined in claim 23. Preferred embodiments of the device and of the method are defined in the dependent claims.

The device and methods of the present invention allow continuous sampling of a flowing liquid. A continuous sample integrates properties of the fluid over a significant part or time of the fluid flow. Preferably, the sample is collected over all or essentially all of the flow. Thus the device and methods of the present invention avoids the problems associated with non-continuous sampling of a liquid flow having physical properties that can vary during the flow. The sample collected is thus more representative of the entire liquid flow.

### BRIEF DESCRIPTION OF THE DRAWINGS

The present invention will be understood and appreciated more fully from the following detailed description, taken in conjunction with the drawings in which:

Fig. 1 is a simplified schematic illustration of milk collection and sampling apparatus, constructed and operative in accordance with a preferred embodiment of the present invention;
Fig. 2 is a sectional side view of a milk sampler constructed and operative in accordance with a preferred embodiment of the present invention;
Fig. 3 is a cross sectional view of the milk sampler of Fig. 2 along the axis A-A;
Fig. 4 is a cross sectional view of the milk sampler of Fig. 2 along the axis B-B;
Fig. 5 is an isometric sectional view of the milk sampler of Fig. 2;
Fig. 6 is an isometric view of a milk sampler constructed and operative in accordance with a preferred embodiment of the present invention;
Fig. 7 is an end view of the milk sampler of Fig. 6;

Fig. 8 is a sectional side view of the milk sampler of Fig. 6;

Fig. 9 is a cross sectional view of the milk sampler of Fig. 7 along the axis D-D;

Fig. 10 is a cross sectional view of the milk sampler of Fig. 7 along the axis E-E;

Fig. 11 is a graph showing sampling ratio A as a function of internal diameter $R_1$ of an annular channel, for a number of different sampling channel diameters;

Fig. 12 is a graph showing deviation from the actual percentage fat content of milk samples collected using a prior art milk sampling device, as a function of the actual percentage fat content of the milk; and

Fig. 13 is a graph showing deviation from the actual percentage fat content of milk samples collected using a milk sampling device constructed and operative in accordance with a preferred embodiment of the invention, as a function of the actual percentage fat content.

## DETAILED DESCRIPTION OF PREFERRED EMBODIMENTS

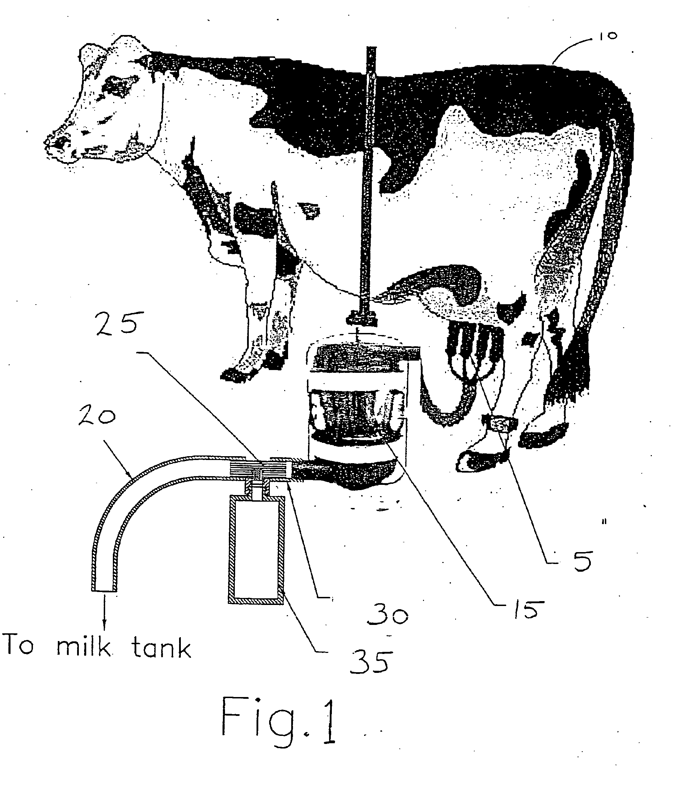

Reference is now made to Fig. 1, which is a simplified schematic illustration of milk collection and sampling apparatus, constructed and operative in accordance with a preferred embodiment of the present invention. As shown, a cluster or claw 5 comprising suction cups are attached to the teats of a cow 10, and the milk passes through milk meter 15 and pipe 20 before collection in a milk tank (not shown). Milk is collected under negative pressure, induced by a vacuum pump (not shown). A milk sampler 25 constructed and operative according to a preferred embodiment of the present invention, located between milk meter 15 and the milk tank, is attached to milk meter 15 *via* connector 30, and sampled milk is collected in sample collection container 35.

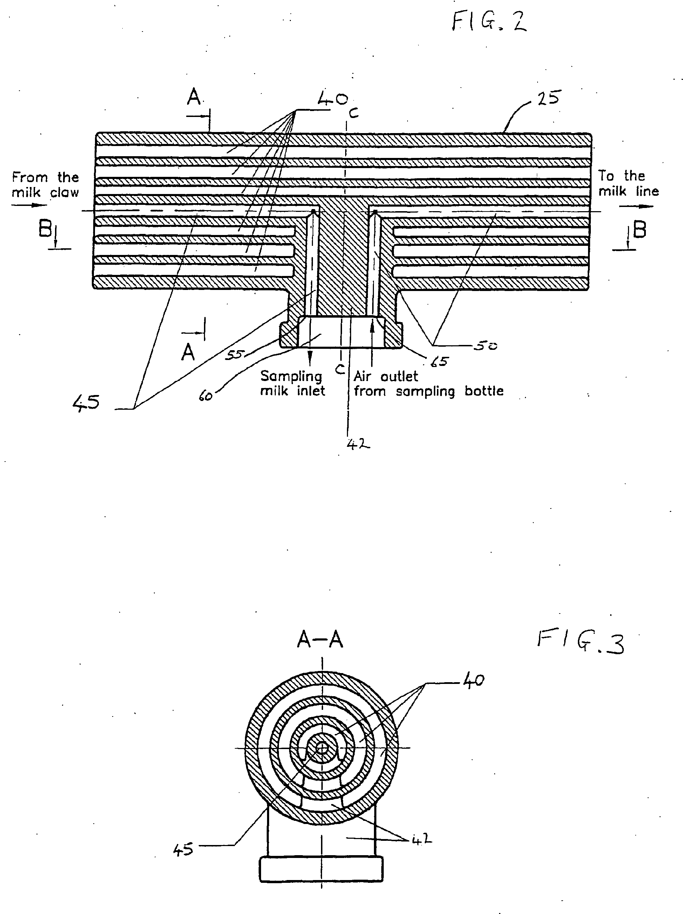

Reference is now made to Fig. 2 which is a sectional side view of milk sampler 25 constructed and operative in accordance with a preferred embodiment of the present invention. Sampler 25 has a generally cylindrical body formed to allow attachment to tubes such as a tube carrying milk from milk claw 5, or from milk meter 15, or tube 20 carrying milk to a milk tank. Sampler 25 comprises a main flow channel which is preferably comprised of a plurality of separate, concentric co-axial, annular channels 40, traversing the length of the sampler from an inlet port to an outlet port.

Sampler 25 also comprises side section 42 which extends within the cylindrical body and which preferably terminates at a sample port outside the cylindrical body.

Sampler 25 also comprises sampling channel 45 which connects the inlet port end of sampler 25 to sample port of the side section 42. At its inlet port end, sampling channel 45 preferably is the centermost of the concentric, annular channels throughout sampler 25.

Sampling channel 45 does not traverse the length of the sampler 25 as do channels 40. Preferably, towards the center of the length of sampler 25, sampling channel 45 bends away from the direction of main flow channel 40, into side section 42, and its sample delivery end exits side section 42 of sampler 25 as sampling inlet 55. The sample port is preferably connected via connector 60 to sample collection container 35. The bend in sampling channel 45 is preferably essentially perpendicular to the direction of direction of main flow channel 40.

Sampler 25 also comprises air-release channel 50 which connects the outlet port end of sampler 25 with the sample port of the side section 42. Air release channel 50 is preferably a symmetrical mirror image to sampling channel 45 about central transverse axis C-C of sampler 25. At its outlet port end, air release channel 50 preferably is the centermost of the concentric, annular channels throughout sampler 25. Preferably, towards the center of the length of sampler 25, air release channel 50 bends into side section 42, and the sample port end of air release channel 50 exits side section 42 of sampler 25 as air outlet 65 from the sample collection container 35 via connector 60.

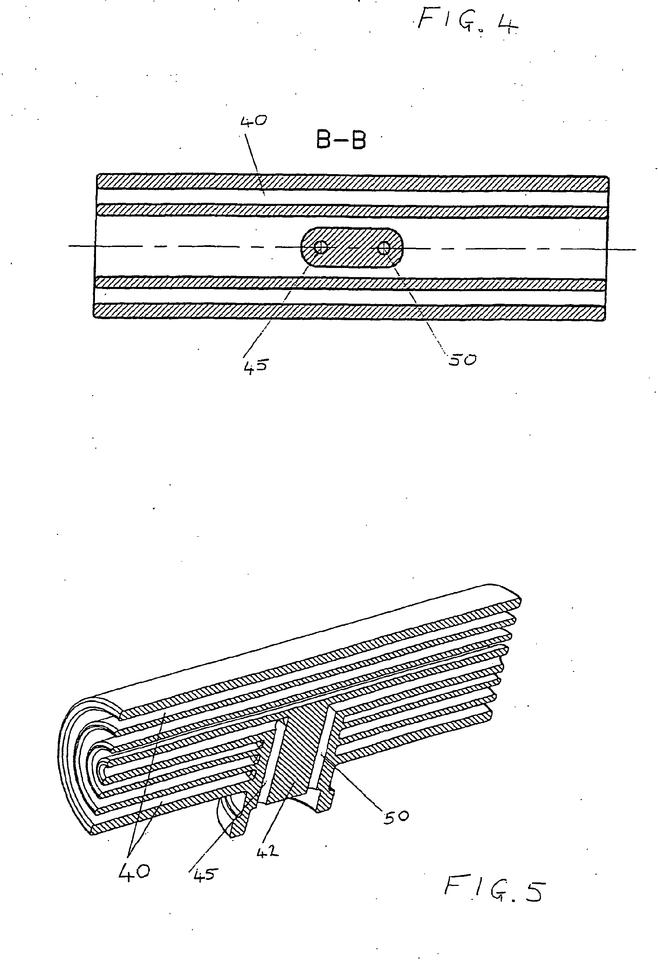

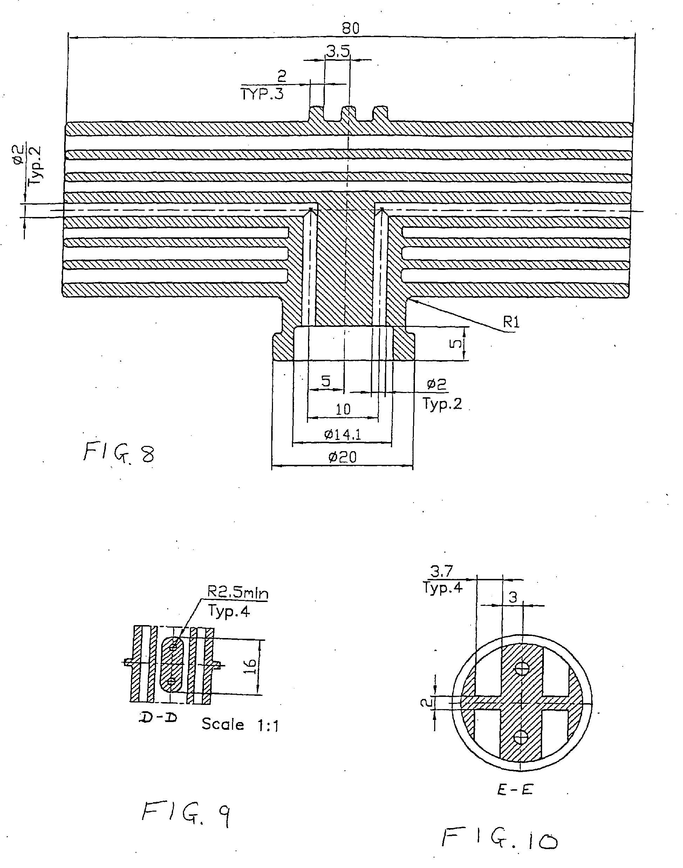

The dimensions of the milk sampling channel 45 and the air-release channel 50 relative to the dimensions of the main flow channels 40, are preferably constructed such that a predetermined percentage of the milk is sampled, *i.e.* a desired proportion of the milk flows through milk sampling channel 45 while the remainder flows through main flow channels 40 to the milk tank. The sampler as shown and described herein with reference to Figs 2-10 is preferably constructed to sample about 1% of the milk passing through sampler 25. The proportion of milk sampled preferably is not influenced or affected to an appreciable extent by changes in the velocity, density temperature or viscosity of the milk.

In a preferred embodiment the internal diameter of the concentric co-axial, annular channels 40 decreases towards the center of the sampler 25.

Reference is now made to Figs. 3-5 which are a cross sectional view of the device of Fig. 2 along the axis A-A; a cross sectional view of the device of Fig. 2 along the axis B-B; and an isometric. sectional view of the device of Fig. 2, respectively. Further details of a preferred construction of sampler 25 can be ascertained from Figs 3-5.

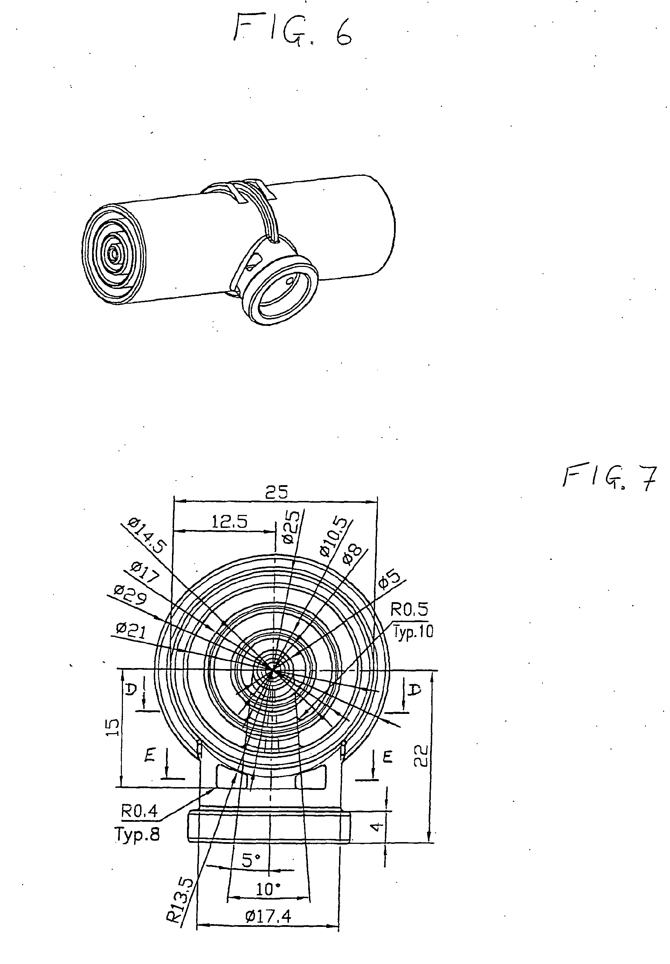

Reference is now made to Fig. 6 which is an isometric view of a milk sampler 25 constructed and operative in accordance with a preferred embodiment of the present invention. Reference is now made to Figs. 7-10 which are a cross sectional view of the milk sampler of Fig. 6; a sectional side view of the milk sampler of Fig. 6; a cross sectional view of the milk sampler of Fig. 7 along the axis D-D; and a cross sectional view of the milk sampler of Fig. 7 along the axis E-E, respectively. Further details of a preferred construction of sampler 25 can be ascertained from Figs. 7-10.

The optimum dimensions of the channels of the body of the sampler are determined by two criteria:

(i) In order to achieve similar hydraulic flow conditions in any two channels, it is necessary to establish similar Reynolds numbers for the flow in those two channels. From known relationships for flow conditions within annular tubes, the following expression is obtained for the diameter $d_s$, of the sampling tube:

$$d_s = \sqrt[3]{\frac{(R_2^4 - R_1^4) + \frac{(R_2^2 - R_1^2)^2}{Ln\,(R_1\,/\,R_2)}}{0.25\,(R_1 + R_2)}}$$

where $R_1$ and $R_2$ are the inner and outer radii respectively of the annular channel.
(ii) The second criterion is the achievement of the desired sampling ratio between the fluid passing into the sampling tube, and that passing down the annular main channels.

The division of the main flow into a number of separate annular flow channels ensures that laminar flow is maintained in each of those annular channels, as well as in the sampling channel. Consequently, the sampling ratio can be calculated according to laminar flow calculations. When these calculations are performed, the following expression is obtained for the ratio, A, between the flow in a specific annular channel and in the sampling tube:

$$A = \frac{(R_2^4 - R_1^4) + \frac{(R_2^2 - R_1^2)^2}{Ln(R_1\,/\,R_2)}}{0.125d_s^4}$$

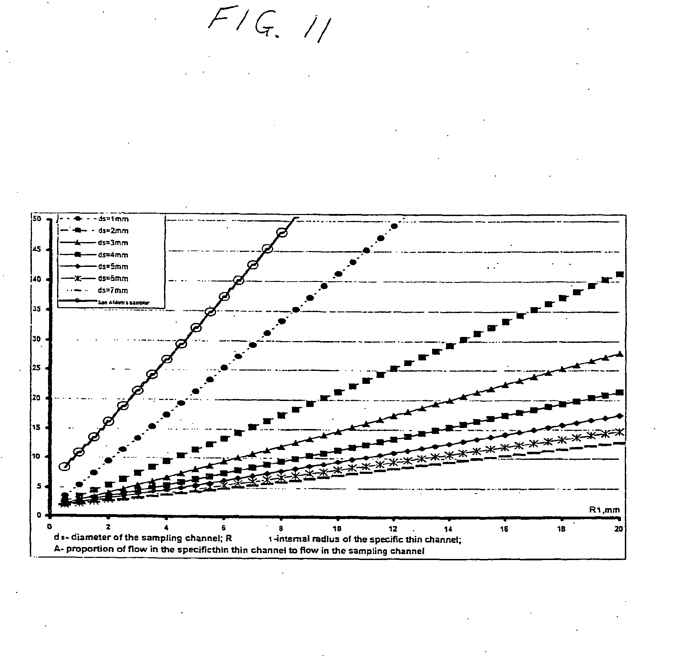

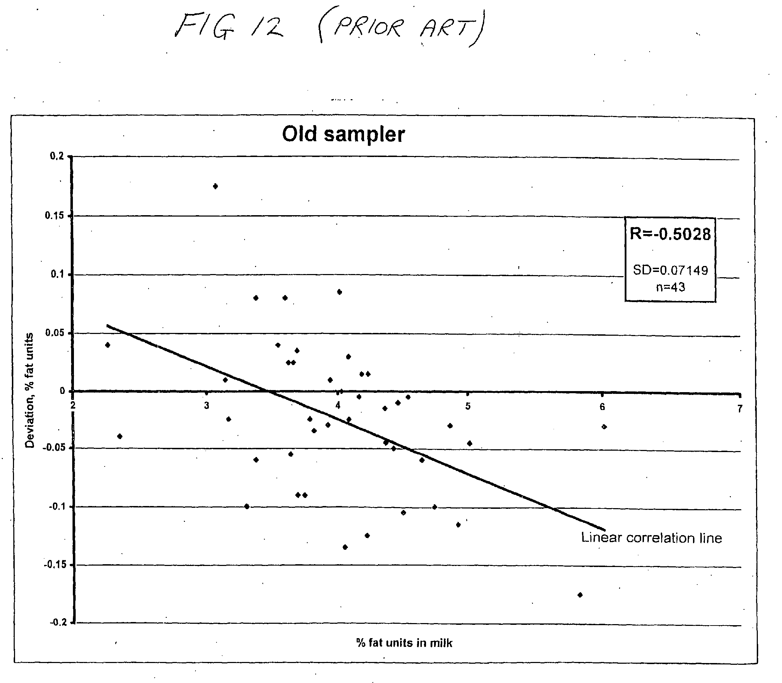

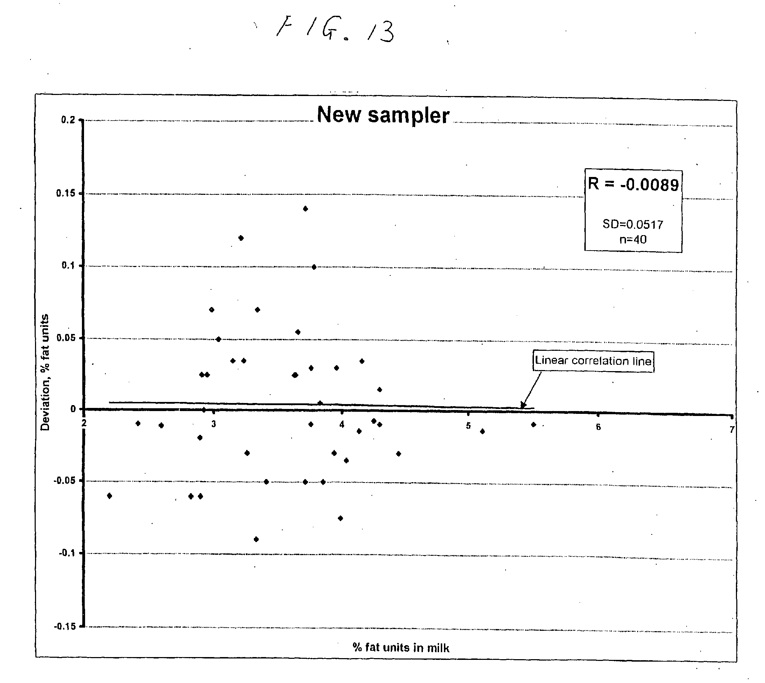

Reference is now made to Fig. 11 which is a graph of the sampling ratio A as a function of the internal diameter $R_1$ of an annular channel, for a number of different sampling channel diameters, according to the expression given in the above equation. The final sampling ratio of the sampler is determined by adding the ratios of all of the individual annular channels.
The milk sampling device constructed and operative in accordance with a preferred embodiment of the invention allows collection of a fluid sample which is more representative of the entire fluid flow than samples collected using prior art sampling devices.
Reference is now made to Fig. 12 which is a graph showing deviation from the actual percentage fat content in milk as a function of the actual percentage fat content, based upon 43 samples collected using a prior art milk sampling device. As shown, the prior art sampling device does not provide a good correlation between the measured percentage of milk fat and the actual percentage (R = -0.5028).
Reference is now made to Fig. 13 which is a graph showing the deviation from the actual percentage fat content in milk, as a function of the actual percentage fat content, based upon 40 samples collected using a milk sampling device constructed and operative in accordance with a preferred embodiment of the invention. As shown, the sampler provides a very good correlation between the measured percentage of milk fat and the actual percentage (R = -0.0089). The graph of Figs. 12 and 13 were plotted, and the linear correlation lines determined, using Microsoft Excel.
Although the invention has described above with relation to sampling of milk, it is appreciated that the sampling device and method is applicable to any liquid, and not just milk. The methods and apparatus of the present invention as described hereinabove is particularly suitable for collecting a continuous sample of non-homogenous fluids. Other liquids and applications of this invention include sampling beer in the brewing industry, sampling from fermenter vats in the pharmaceutical industry etc.

**Claims**

1. A liquid sampling device (25) comprising:

a cylindrical body comprising an inlet port and an outlet port;
a side section (42) comprising a sampling port; and
a sampling channel (45), having an inlet port end and a sampling port end, the sampling channel (45) connecting the inlet port to the sampling port, the sampling channel (45) being disposed at its inlet port end essentially at the center of the cylindrical body and at its sampling port end in the side section (42);
**characterized in that**
the cylindrical body comprises a plurality of concentric annular channels (40) traversing the cylindrical body connecting the inlet port and the outlet port; and
the liquid sampling device (25) comprises an air channel (50), connecting the sampling port to the outlet port, the air channel (50) being disposed at its outlet port end essentially at the center of the cylindrical body, and its sampling port end in the side section (42).

2. A liquid sampling device (25) according to claim 1 wherein the side section (42) is essentially perpendicular to the cylindrical body.

3. A liquid sampling device (25) according to any of the preceding claims wherein the side section (42) extends within the cylindrical body.

4. A liquid sampling device (25) according to any of the preceding claims wherein the side section (42) extends outwards past the outer circumference of the cylindrical body.

5. A liquid sampling device (25) according to any of the preceding claims wherein the sampling channel (45) bends between its inlet port end and its sampling port end.

6. A liquid sampling device (25) according to claim 5 wherein the sampling channel (45) bends essentially perpendicularly to the cylindrical body.

7. A liquid sampling device (25) according to any of the preceding claims wherein the air channel (50) bends between its outlet port end and its sampling port end.

8. A liquid sampling device (25) according to claim 7 wherein the air channel (50) bends essentially perpendicularly to the cylindrical body.

9. A liquid sampling device (25) according to any of the preceding claims wherein the sampling channel (45) and the air channel (50) are essentially symmetrical about a plane perpendicular to the cylindrical body.

10. A liquid sampling device (25) according to any of the preceding claims which is symmetrical about a plane perpendicular to the cylindrical body.

11. A liquid sampling device (25) according to any of the preceding claims wherein the ratio of the dimensions of the plurality of concentric annular channels (40) and the dimensions of the sampling channel (45) give predetermined sample percentage.

12. A liquid sampling device (25) according to claim 11 wherein the sample percentage is about 1%.

13. A liquid sampling device (25) according to any of the preceding claims wherein the internal diameter of each of the plurality of concentric annular channels (40) increases towards the external periphery of the cylindrical body.

14. A liquid sampling device (25) according to any of the preceding claims wherein the internal diameter of the sampling channel (45) is less than the internal diameter of any of the plurality of concentric annular channels (40).

15. A liquid sampling device (25) according to any of the preceding claims wherein the plurality of concentric annular channels (40) comprises three concentric annular channels (40).

16. A liquid sampling device (25) according to any of the preceding claims wherein the inlet port is connected to a liquid source.

17. A liquid sampling device (25) according to any of the preceding claims wherein the sampling port is connected to a sample collection container (35).

18. A liquid sampling device (25) according to any of the preceding claims wherein the outlet port is connected to a liquid collection container.

19. A liquid sampling device (25) according to any of the preceding claims wherein about 1% of the liquid entering the inlet port enters the sampling channel (45).

20. A liquid sampling device (25) according to any of the preceding claims wherein liquid entering the plurality of concentric annular channels (40) at the inlet port exits the device at the outlet port, and wherein liquid entering the sampling channel (45) at the inlet port exits the device (25) at the sampling port.

21. A liquid sampling device (25) according to claim 20 wherein the composition of the liquid exiting the device at the sampling port is representative of the composition of the liquid entering the device at the inlet port.

22. A liquid sampling device (25) according to any of the preceding claims wherein the liquid is milk.

23. Method of essentially continuously sampling a liquid flow comprising the steps of:

   flowing the liquid through a device (25); and
   sampling a predetermined fraction of the liquid through a sampling channel (45);
   **characterized in that**
   the device (25) comprises a plurality of concentric annular channels (40); and
   the sampling channel (45) is disposed centermost to the concentric annular channels (40).

24. A method according to claim 23 and also comprising the step of collecting the liquid flowing through the plurality of concentric annular channels (40).

25. A method according to any of the preceding claims 23-24 and also comprising the step of collecting the sampled liquid flowing through the sampling channel (45).

26. A method according to any of preceding claims 23-25 wherein the device comprises:

   a cylindrical body comprising an inlet port and an outlet port, wherein the plurality of concentric annular channels (40) traverse the cylindrical body connecting the inlet port and the outlet port; a side section (42) comprising a sampling port, wherein the sampling channel (45) connects the inlet port to the sampling port, the sampling channel (45) having an inlet port end and a sampling port end, the sampling channel (45) being disposed at its inlet port end essentially at the center of the cylindrical body and at its sampling port end in the side section (42); and an air channel (50) connecting the sampling port to the outlet port, the air channel (50) being disposed at its outlet port end essentially at the center of the cylindrical body, and its sampling port end in the side section (42).

27. A method according to any of preceding claims 23-26 wherein the predetermined fraction is predetermined based upon the ratio of the dimensions of the plurality of concentric annular channels (40) and the dimensions of the sampling channel (45).

28. A method according to any of preceding claims 23-27 wherein the predetermined fraction is about 1%.

29. A method according to any of preceding claims 23-28 wherein the liquid flow is non-homogenous and the sampled liquid is essentially representative of the liquid flow.

30. A method according to any of preceding claims 23-29 wherein the composition of the sampled liquid is essentially representative of the composition of the liquid flow.

31. A method according to any of preceding claims 23-30 wherein the liquid is milk.

32. A method according to claim 31 wherein the lipid content of the milk is lower at the start of the milk flow, and the lipid content of the sampled milk is essentially representative of the overall lipid content of the entire milk flow.

**Patentansprüche**

1. Eine Vorrichtung zur Entnahme flüssiger Proben (25) mit:

einem zylindrischen Körper mit einem Einlassanschluss und einem Auslassanschluss, einem Seitenabschnitt (42) mit einem Probenentnahmeanschluss, und einem Probenentnahmekanal (45), der ein Einlassanschlussende und ein Probenentnahmeanschlussende besitzt, wobei der Probenentnahmekanal (45) den Einlassanschluss mit dem Probenentnahmeanschluss verbindet, wobei der Probenentnahmekanal (45) an seinem Einlassanschlussende im wesentlichen in der Mitte des zylindrischen Körpers und an seinem Probenentnahmeanschlussende in dem Seitenabschnitt (42) angeordnet ist,

**dadurch gekennzeichnet, dass**

der zylindrische Körper mehrere konzentrische ringförmige Kanäle (40) aufweist, die den zylindrischen Körper durchlaufen und den Einlassanschluss und den Auslassanschluss verbinden, und

die Vorrichtung zur Entnahme flüssiger Proben (25) einen Luftkanal (50) aufweist, der den Probenentnahmeanschluss mit dem Auslassanschluss verbindet, wobei der Luftkanal (50) an seinem Auslassanschlussende im wesentlichen in der Mitte des zylindrischen Körpers und an seinem Probenentnahmeanschlussende in dem Seitenabschnitt (42) angeordnet ist.

2. Eine Vorrichtung zur Entnahme flüssiger Proben (25) gemäß Anspruch 1, wobei der Seitenabschnitt (42) im wesentlichen senkrecht zu dem zylindrischen Körper ist.

3. Eine Vorrichtung zur Entnahme flüssiger Proben (25) gemäß einem der vorstehenden Ansprüche, wobei der Seitenabschnitt (42) sich in dem zylindrischen Körper erstreckt.

4. Eine Vorrichtung zur Entnahme flüssiger Proben (25) gemäß einem der vorstehenden Ansprüche, wobei der Seitenabschnitt (42) sich nach außen über den Außenumfang des zylindrischen Körpers hinaus erstreckt.

5. Eine Vorrichtung zur Entnahme flüssiger Proben (25) gemäß einem der vorstehenden Ansprüche, wobei der Probenentnahmekanal (45) zwischen seinem Einlassanschlussende und seinem Probenentnahmeanschlussende abgebogen ist.

6. Eine Vorrichtung zur Entnahme flüssiger Proben (25) gemäß einem der vorstehenden Ansprüche, wobei der Probenentnahmekanal (45) im wesentlichen senkrecht zu dem zylindrischen Körper abgebogen ist.

7. Eine Vorrichtung zur Entnahme flüssiger Proben (25) gemäß einem der vorstehenden Ansprüche, wobei der Luftkanal (50) zwischen seinem Auslassanschlussende und seinem Probenentnahmeanschlussende abgebogen ist.

8. Eine Vorrichtung zur Entnahme flüssiger Proben (25) gemäß Anspruch 7, wobei der Luftkanal (50) im wesentlichen senkrecht zu dem zylindrischen Körper abgebogen ist.

9. Eine Vorrichtung zur Entnahme flüssiger Proben (25) gemäß einem der vorstehenden Ansprüche, wobei der Probenentnahmekanal (45) und der Luftkanal (50) im wesentlichen symmetrisch zu einer Ebene senkrecht zu dem zylindrischen Körper sind.

10. Eine Vorrichtung zur Entnahme flüssiger Proben (25) gemäß einem der vorstehenden Ansprüche, die zu einer Ebene senkrecht zu dem zylindrischen Körper symmetrisch ist.

11. Eine Vorrichtung zur Entnahme flüssiger Proben (25) gemäß einem der vorstehenden Ansprüche, wobei das Verhältnis der Abmessungen der mehreren konzentrischen ringförmigen Kanäle (40) zu den Abmessungen des Probenentnahmekanals (45) einen vorbestimmten Probenanteil vorgibt.

12. Eine Vorrichtung zur Entnahme flüssiger Proben (25) gemäß Anspruch 11, wobei der Probenanteil etwa 1% trägt.

13. Eine Vorrichtung zur Entnahme flüssiger Proben (25) gemäß einem der vorstehenden Ansprüche, wobei der Innendurchmesser jedes der mehreren konzentrischen ringförmigen Kanäle (40) zu dem externen Umfang des zylindrischen Körpers hin zunimmt.

14. Eine Vorrichtung zur Entnahme flüssiger Proben (25) gemäß einem der vorstehenden Ansprüche, wobei der Innendurchmesser des Probenentnahmekanals (45) geringer ist als der Innendurchmesser irgendeines der mehreren konzentrischen ringförmigen Kanäle (40).

15. Eine Vorrichtung zur Entnahme flüssiger Proben (25) gemäß einem der vorstehenden Ansprüche, wobei die mehreren konzentrischen ringförmigen Kanäle (40) drei konzentrische ringförmige Kanäle (40) umfassen.

16. Eine Vorrichtung zur Entnahme flüssiger Proben (25) gemäß einem der vorstehenden Ansprüche, wobei der Einlassanschluss mit einer Flüssigkeitsquelle verbunden ist.

17. Eine Vorrichtung zur Entnahme flüssiger Proben (25) gemäß einem der vorstehenden Ansprüche, wobei der Probenentnahmeanschluss mit einem Probensammelbehälter (35) verbunden ist.

18. Eine Vorrichtung zur Entnahme flüssiger Proben (25) gemäß einem der vorstehenden Ansprüche, wobei der Auslassanschluss mit einem Flüssigkeitssammelbehälter verbunden ist.

19. Eine Vorrichtung zur Entnahme flüssiger Proben (25) gemäß einem der vorstehenden Ansprüche, wobei etwa 1% der Flüssigkeit, die in den Einlassanschluss eintritt, in den Probenentnahmekanal (45) eintritt.

20. Eine Vorrichtung zur Entnahme flüssiger Proben (25) gemäß einem der vorstehenden Ansprüche, wobei Flüssigkeit, die in die mehreren konzentrischen ringförmigen Kanäle (40) an dem Einlassanschluss eintritt, die Vorrichtung an dem Auslassanschluss verlässt, und wobei Flüssigkeit, die in den Probenentnahmekanal (45) an dem Einlassanschluss eintritt, die Vorrichtung (25) an den Probenentnahmeanschluss verlässt.

21. Eine Vorrichtung zur Entnahme flüssiger Proben (25) gemäß Anspruch 20, wobei die Zusammensetzung der Flüssigkeit, die die Vorrichtung an dem Probenentnahmeanschluss verlässt, repräsentativ für die Zusammensetzung der Flüssigkeit ist, die in die Vorrichtung an dem Einlassanschluss eintritt.

22. Eine Vorrichtung zur Entnahme flüssiger Proben (25) gemäß einem der vorstehenden Ansprüche, wobei die Flüssigkeit Milch ist.

23. Ein Verfahren zum im wesentlichen kontinuierlichen Entnehmen einer Probe aus einem Flüssigkeitsstrom mit den Schritten:

Strömenlassen der Flüssigkeit durch eine Vorrichtung (25), und
Entnehmen eines vorbestimmten Anteils der Flüssigkeit durch einen Probenentnahmekanal (45),
**dadurch gekennzeichnet, dass**
die Vorrichtung (25) mehrere konzentrische ringförmige Kanäle (40) aufweist, und
der Probenentnahmenkanal (45) im Zentrum der konzentrischen ringförmigen Kanäle (40) angeordnet ist.

24. Ein Verfahren gemäß Anspruch 23, ferner mit dem Schritt des Sammelns der durch die mehreren konzentrischen ringförmigen Kanäle (40) strömenden Flüssigkeit.

25. Ein Verfahren gemäß einem der vorstehenden Ansprüche 23 bis 24, ferner mit dem Schritt des Sammelns der entnommenen Flüssigkeit, die durch den Probenentnahmekanal (45) strömt.

26. Ein Verfahren gemäß einem der vorstehenden Ansprüche 23 bis 25, wobei die Vorrichtung umfasst:

einen zylindrischen Körper mit einem Einlassanschluss und einem Auslassanschluss, wobei die mehreren konzentrischen ringförmigen Kanäle (40) den zylindrischen Körper durchlaufen und den Einlassanschluss und den Auslassanschluss verbinden,

einen Seitenabschnitt (42), der einen Probenentnahmeanschluss aufweist, wobei der Probenentnahmekanal (45) den Einlassanschluss mit dem Probenentnahmeanschluss verbindet, wobei der Probenentnahmekanal (45) ein Einlassanschlussende und ein Probenentnahmeanschlussende besitzt, wobei der Probenentnahmekanal (45) an seinem Einlassanschlussende im wesentlichen in der Mitte des zylindrischen Körpers und an seinem Probenentnahmeanschlussende in dem Seitenabschnitt (42) angeordnet ist, und

einen Luftkanal (50), der den Probenentnahmeanschluss mit dem Auslassansschluss verbindet, wobei der Luftkanal (50) an seinem Auslassanschlussende im wesentichen in der Mitte des zylindrischen Körpers und an seinem Probenentnahmeanschlussende in dem Seitenabschnitt (42) angeordnet ist.

27. Ein Verfahren gemäß einem der vorstehenden Ansprüche 23 bis 26, wobei der vorbestimmte Anteil vorbestimmt wird basierend auf dem Verhältnis der Abmessungen der mehreren konzentrischen ringförmigen Kanäle (40) zu den Abmessungen des Probenentnahmekanals (45).

28. Ein Verfahren gemäß einem der vorstehenden Ansprüche 23 bis 27, wobei der vorbestimmte Anteil etwa 1% beträgt.

29. Ein Verfahren gemäß einem der vorstehenden Ansprüche 23 bis 28, wobei der Flüssigkeitsstrom nicht homogen und die entnommene Flüssigkeit im wesentlichen repräsentativ für den Flüssigkeitsstrom ist.

30. Ein Verfahren gemäß einem der vorstehenden Ansprüche 23 bis 29, wobei die Zusammensetzung der entnommenen Flüssigkeit im wesentlichen repräsentativ für die Zusammensetzung des Flüssigkeitsstroms ist.

31. Ein Verfahren gemäß einem der vorstehenden Ansprüche 23 bis 30, wobei die Flüssigkeit Milch ist.

32. Ein Verfahren gemäß Anspruch 31, wobei der Lipidgehalt der Milch niedriger ist zu Beginn des Milchstroms und der Lipidgehalt der entnommenen Milch im wesentlichen repräsentativ ist für den Gesamtlipidgehalt des gesamten Milchstroms.

## Revendications

1. Dispositif (25) d'échantillonnage pour un liquide comprenant :

   un corps cylindrique comprenant un orifice d'entrée et un orifice de sortie ;
   une section (42) latérale comprenant un orifice d'échantillonnage ; et
   un canal (45) d'échantillonnage ayant une extrémité d'orifice d'entrée et une extrémité d'orifice d'échantillonnage, le canal (45) d'échantillonnage mettant l'orifice d'entrée en communication avec l'orifice d'échantillonnage, le canal (45) d'échantillonnage étant disposé à son extrémité d'orifice d'entrée sensiblement au centre du corps cylindrique et à son extrémité d'orifice d'échantillonnage dans la section (42) latérale ;

   **caractérisé en ce que**

   le corps cylindrique comprend une pluralité de canaux (40) annulaires concentriques traversant le corps cylindrique en mettant l'orifice d'entrée et l'orifice de sortie en communication ; et le dispositif (25) d'échantillonnage pour un liquide comprend un canal (50) pour de l'air mettant l'orifice d'échantillonnage en communication avec l'orifice de sortie, le canal (50) pour de l'air étant disposé à son extrémité d'orifice de sortie sensiblement au centre du corps cylindrique et à son extrémité d'orifice d'échantillonnage dans la section (42) latérale.

2. Dispositif (25) d'échantillonnage pour un liquide suivant la revendication 1, dans lequel la section (42) latérale est sensiblement perpendiculaire au corps cylindrique.

3. Dispositif (25) d'échantillonnage pour un liquide suivant l'une quelconques des revendications précédentes, dans lequel la section (42) latérale s'étend dans le corps cylindrique.

4. Dispositif (25) d'échantillonnage pour un liquide suivant l'une quelconques des revendications précédentes, dans lequel la section (42) latérale s'étend à l'extérieur au-delà de la circonférence extérieure du corps cylindrique.

5. Dispositif (25) d'échantillonnage pour un liquide suivant l'une quelconques des revendications précédentes, dans lequel le canal (45) d'échantillonnage se courbe entre son extrémité d'orifice d'entrée et son extrémité d'orifice d'échantillonnage.

6. Dispositif (25) d'échantillonnage pour un liquide suivant la revendication 5, dans lequel le canal (45) d'échantillonnage se courbe sensiblement perpendiculairement au corps cylindrique.

7. Dispositif (25) d'échantillonnage pour un liquide suivant l'une quelconques des revendications précédentes, dans lequel le canal (50) pour de l'air se courbe entre son extrémité d'orifice de sortie et son extrémité d'orifice d'échantillonnage.

8. Dispositif (25) d'échantillonnage pour un liquide suivant la revendication 7, dans lequel le canal (50) pour de l'air se courbe sensiblement perpendiculairement au corps cylindrique.

9. Dispositif (25) d'échantillonnage pour un liquide suivant l'une quelconques des revendications précédentes, dans lequel le canal (45) d'échantillonnage et le canal (50) pour de l'air sont sensiblement symétriques par rapport à un plan perpendiculaire au corps cylindrique.

10. Dispositif (25) d'échantillonnage pour un liquide suivant l'une quelconques des revendications précédentes, qui est symétrique par rapport à un plan perpendiculaire au corps cylindrique.

11. Dispositif (25) d'échantillonnage pour un liquide suivant l'une quelconques des revendications précédentes, dans lequel le rapport des dimensions de la pluralité de canaux (40) annulaires concentriques et des dimensions du canal (45) d'échantillonnage donne un pourcentage de l'échantillon déterminé à l'avance.

12. Dispositif (25) d'échantillonnage pour un liquide suivant la revendication 11, dans lequel le pourcentage de l'échantillon est d'environ 1%.

13. Dispositif (25) d'échantillonnage pour un liquide suivant l'une quelconques des revendications précédentes, dans lequel le diamètre intérieur de chacun de la pluralité des canaux (40) annulaires concentriques augmente en allant vers la périphérie extérieure du corps cylindrique.

14. Dispositif (25) d'échantillonnage pour un liquide suivant l'une quelconques des revendications précédentes, dans lequel le diamètre intérieur du canal (45) d'échantillonnage est plus petit que le diamètre intérieur de n'importe lequel de la pluralité de canaux (40) annulaires concentriques.

15. Dispositif (25) d'échantillonnage pour un liquide suivant l'une quelconques des revendications précédentes, dans lequel la pluralité de canaux (40) annulaires concentriques comprend trois canaux (40) annulaires concentriques.

16. Dispositif (25) d'échantillonnage pour un liquide suivant l'une quelconques des revendications précédentes, dans lequel l'orifice d'entrée communique avec une source de liquide.

17. Dispositif (25) d'échantillonnage pour un liquide suivant l'une quelconques des revendications précédentes, dans lequel l'orifice d'échantillonnage communique avec un récipient (35) de recueil d'échantillon.

18. Dispositif (25) d'échantillonnage pour un liquide suivant l'une quelconques des revendications précédentes, dans lequel l'orifice de sortie communique avec un récipient de recueil de liquide.

19. Dispositif (25) d'échantillonnage pour un liquide suivant l'une quelconques des revendications précédentes, dans lequel environ 1% du liquide entrant dans l'orifice d'entrée entre dans le canal (45) d'échantillonnage.

20. Dispositif (25) d'échantillonnage pour un liquide suivant l'une quelconques des revendications précédentes, dans lequel du liquide entrant dans la pluralité de canaux (40) annulaires concentriques à l'orifice d'entrée sort du dispositif à l'orifice de sortie et dans lequel du liquide entrant dans le canal (45) d'échantillonnage à l'orifice d'entrée sort du dispositif (25) à l'orifice d'échantillonnage.

21. Dispositif (25) d'échantillonnage pour un liquide suivant la revendication 20, dans lequel la composition du liquide sortant du dispositif à l'orifice d'échantillonnage est représentative de la composition du liquide entrant dans le dispositif à l'orifice d'entrée.

22. Dispositif (25) d'échantillonnage pour un liquide suivant l'une quelconques des revendications précédentes, dans lequel le liquide est du lait.

23. Procédé d'échantillonnage sensiblement continu d'un courant de liquide comprenant les stades dans desquels :

on fait s'écouler le liquide dans un dispositif (25) ; et
on échantillonne une fraction déterminée du liquide passant dans un canal (45) d'échantillonnage ;
**caractérisé en ce que**
le dispositif (25) comprend une pluralité de canaux (40) annulaires concentriques ; et
le canal (45) d'échantillonnage est disposé le plus au centre par rapport aux canaux (40) annulaires concentriques.

24. Procédé suivant la revendication 23 et comprenant également le stade de recueil du liquide s'écoulant dans la pluralité de canaux (40) annulaires concentriques.

25. Procédé suivant l'une quelconques des revendications précédentes 23 et 24 et comprenant aussi le stade de recueil du liquide échantillonné s'écoulant dans le canal (45) d'échantillonnage.

26. Procédé suivant l'une quelconques des revendications précédentes 23 à 25 dans lequel le dispositif comprend :

   un corps cylindrique comprenant un orifice d'entrée et un orifice de sortie, la pluralité de canaux (40) annulaires concentriques traversant le corps cylindrique en mettant l'orifice d'entrée et l'orifice de sortie en communication ;
   une section (32) latérale comprenant un orifice d'échantillonnage, le canal (45) d'échantillonnage mettant l'orifice d'entrée en communication avec l'orifice d'échantillonnage, le canal (45) d'échantillonnage ayant une extrémité d'orifice d'entrée et une extrémité d'orifice d'échantillonnage, le canal (45) d'échantillonnage étant disposé à son extrémité d'orifice d'entrée sensiblement au centre du corps cylindrique et à son extrémité d'orifice d'échantillonnage dans la section (42) ; et
   un canal (50) pour de l'air mettant l'orifice d'échantillonnage en communication avec l'orifice de sortie, le canal (50) pour de l'air étant disposé à son extrémité d'orifice de sortie sensiblement au centre du corps cylindrique et à son extrémité d'orifice d'échantillonnage dans la section (42) latérale.

27. Procédé suivant l'une quelconques des revendications précédentes 23 à 26, dans lequel la fraction déterminée à l'avance est déterminée à l'avance sur la base du rapport des dimensions de la pluralité de canaux (40) annulaires concentriques et des dimensions du canal (45) d'échantillonnage.

28. Procédé suivant l'une quelconques des revendications précédentes 23 à 27, dans lequel la fraction déterminée à l'avance est d'environ 1%.

29. Procédé suivant l'une quelconques des revendications précédentes 23 à 28 dans lequel le courant de liquide n'est pas homogène et le liquide échantillonné est sensiblement représentatif du courant de liquide.

30. Procédé suivant l'une quelconques des revendications précédentes 23 à 29 dans lequel la composition du liquide échantillonné est sensiblement représentative de la composition du courant de liquide.

31. Procédé suivant l'une quelconques des revendications précédentes 23 à 30 dans lequel le liquide est du lait.

32. Procédé suivant la revendication 31, dans lequel la teneur en lipide du lait est plus petite au début du courant de lait et la teneur en lipide du lait échantillonné est sensiblement représentative de la teneur global en lipide de tout le courant de lait.

25

20

To milk tank

30

35

5

15

Fig.1

FIG. 2

A

40
C

25

From the
milk claw

To the
milk line

B

B

A

55

50

65

C

Sampling
milk inlet

Air outlet
from sampling bottle

45

60

42

A—A

FIG. 3

40

45

42

FIG. 4

B-B

40

45

50

FIG. 5

40

45

42

50

FIG. 6

FIG. 7

80

3.5

2
TYP.3

ø2
Typ.2

R1

5

5

ø2
Typ.2

10

ø14.1

ø20

FIG. 8

R2.5min
Typ.4

16

D-D    Scale 1:1

FIG. 9

3.7
Typ.4

3

2

E-E

FIG. 10

## FIG. 11

Legend:
- ds=1mm
- ds=2mm
- ds=3mm
- ds=4mm
- ds=5mm
- ds=6mm
- ds=7mm
- See Alarm's sampler

d s - diameter of the sampling channel; R 1 -internal radius of the specific thin channel;
A - proportion of flow in the specific thin thin channel to flow in the sampling channel

FIG 12 (PRIOR ART)

Old sampler

R=-0.5028

SD=0.07149
n=43

Linear correlation line

Deviation, % fat units

% fat units in milk

FIG. 13

New sampler

R = -0.0089

SD=0.0517
n=40

Linear correlation line

Deviation, % fat units

% fat units in milk

EP 1 254 595 B1

**EP 1 254 595 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4147062 A
- US 4574630 A
- US 5388549 A
- US 5572946 A
- US 3803921 A